# FASCICULE DE BREVET EUROPEEN

(11) **EP 2 526 087 B1**
(45) Date de publication et mention de la délivrance du brevet: **29.04.2015**
(21) Numéro de dépôt: 11700923.3
(22) Date de dépôt: 14.01.2011
(51) Int. Cl.: C07C 407/00

(54) **PROCEDE DE FABRICATION D'UN HYDROPEROXYDE D'ALKYLE**
VERFAHREN ZUR HERSTELLUNG VON ALKYLHYDROPEROXID
METHOD FOR PRODUCING ALKYL HYDROPEROXIDE

(30) Priorité: 21.01.2010 FR 1050385
(43) Date de publication de la demande: 28.11.2012
(73) Titulaire: Rhodia Operations, 93306 Aubervilliers (FR)
(72) Inventeur: IGERSHEIM, Françoise, F-69003 Lyon (FR); STREIFF, Stéphane, F-69006 Lyon (FR); VERACINI, Serge, F-69005 Lyon (FR)
(74) Mandataire: Chatelan, Florence Anne
(86) Numéro de dépôt international: PCT/EP2011/050470
(87) Numéro de publication internationale: WO 2011/089075

(56) Documents cités:
- EP-A1- 0 404 417
- FR-A- 1 527 390
- FR-A1- 2 291 956
- GB-A- 816 200
- US-A- 3 479 394
- US-A- 3 719 706

## Description

La présente invention concerne un procédé de préparation d'un hydroperoxyde d'alkyle obtenu par oxydation par l'oxygène d'un hydrocarbure saturé.

Elle concerne plus particulièrement la production d'hydroperoxyde de cyclohexyle obtenu par oxydation du cyclohexane avec de l'oxygène moléculaire ou un gaz contenant de l'oxygène moléculaire, de préférence en l'absence de catalyseur.

La production d'hydroperoxyde de cyclohexyle est généralement la première étape du procédé de fabrication de cyclohexanone et/ou cyclohexanol.

Ces composés cyclohexanone et cyclohexanol seuls ou en mélanges sont des matières premières importantes pour la production d'acide adipique et d'epsilon-caprolactame, monomères pour la fabrication des matériaux thermoplastiques polyamides, ou dans la synthèse des matériaux polyuréthannes, polyesters ou analogues.

En effet, le principal procédé de fabrication de l'acide adipique utilisé à l'échelle industrielle, consiste à oxyder par l'oxygène moléculaire, le cyclohexane en alcool et/ou cétone. Le mélange de cétone et alcool est ensuite oxydé en acide adipique par l'acide nitrique.

Dans un des modes de réalisation du procédé d'oxydation du cyclohexane en alcool/cétone, le cyclohexane est oxydé dans une première étape, en présence ou non de catalyseur principalement en hydroperoxyde de cyclohexyle, cet hydroperoxyde étant transformé en alcool/cétone dans une seconde étape. Cette première étape d'oxydation du cyclohexane est généralement réalisée en milieu biphasique gaz/liquide, le gaz oxydant, de l'oxygène ou un gaz contenant de l'oxygène, est introduit dans le milieu liquide dans des réacteurs constitués d'une ou plusieurs colonnes à bulles compartimentées ou non, montées en série lorsque leur nombre est supérieur à un, fonctionnant soit à co-courant, soit à contre-courant du sens de circulation de la phase liquide constituée principalement par du cyclohexane à l'état liquide. Cette étape est notamment décrite dans les brevets GB 777087, 1112837, 964869, 1191573, US 3479394, US 4877903.

Le milieu réactionnel recueilli en sortie de l'étape d'oxydation contient du cyclohexane qui n'a pas réagi et les produits formés au cours de l'oxydation.

Ces produits oxydés peuvent être classés en deux catégories :
- une première catégorie constituée des composés qui sont ou peuvent être transformés en alcool/cétone cyclique dans l'aval du procédé. Dans le cas de l'oxydation du cyclohexane, ces produits dits « olonogènes » sont le cyclohexanol, la cyclohexanone et l'hydroperoxyde de cyclohexyle, et ;
- une seconde catégorie qui comprend les produits oxydés qui ne peuvent pas être transformés en alcool ou cétone cycliques, produits résultant de l'ouverture du cycle hydrocarboné au cours du processus d'oxydation tels des mono ou diacides carboxyliques. Par exemple, toujours dans le cas de l'oxydation du cyclohexane, ces produits oxydés dits « non olonogènes » sont l'acide adipique, des hydroxyacides comme l'acide hydroxy-6 hexanoïque, des hydroperoxyacides comme l'acide hydroperoxy-6 hexanoïque, ou des produits cycliques portant deux fonctions oxygénées comme des hydroxy-cyclohexanols ou hydroxy-cyclohexanones.

Les produits de la première catégorie sont peu solubles dans l'eau. Au contraire, les produits de la seconde catégorie sont solubles dans l'eau et peuvent être extraits par un lavage à l'eau.

Dans les procédés exploités, une partie du cyclohexane qui n'a pas réagi à l'étape d'oxydation est séparée du milieu réactionnel et recyclée dans les réacteurs d'oxydation.

Le milieu réactionnel résultant est ensuite soumis à un lavage ou extraction par l'eau pour séparer les produits solubles dans l'eau et appartenant généralement à la seconde catégorie de produits oxydés.

Le milieu réactionnel ainsi récupéré contient principalement de l'hydroperoxyde de cyclohexyle en solution dans le cyclohexane, avec d'autres produits oxydés, appartenant principalement à la première catégorie décrite ci-dessus.

Cette solution d'hydroperoxyde de cyclohexyle est avantageusement introduite dans une étape de décomposition ou déperoxydation en présence de catalyseur pour transformer l'hydroperoxyde en cétone et alcool.

Le procédé d'extraction à l'eau des produits oxydés appartenant à la seconde catégorie présente certains inconvénients, notamment l'extraction n'est pas parfaitement sélective. Ainsi, des produits oxydés olonogènes, produits appartenant à la première catégorie, sont partiellement extraits par la phase aqueuse, ce qui diminue le rendement et l'économie du procédé.

Un des buts de la présente invention est de remédier à ces inconvénients en proposant une solution permettant notamment de diminuer fortement la quantité de produits oxydés de la première catégorie dans la phase aqueuse et donc d'améliorer l'économie du procédé.

A cet effet, l'invention propose un procédé de fabrication d'hydroperoxyde d'alkyle par oxydation par l'oxygène moléculaire d'un hydrocarbure saturé comprenant les étapes successives suivantes :
❖ oxydation de l'hydrocarbure par l'oxygène,
❖ séparation à partir du milieu réactionnel d'oxydation d'au moins une partie de l'hydrocarbure non oxydé et recyclage de celui-ci dans l'étape d'oxydation,
❖ lavage par de l'eau du milieu réactionnel d'oxydation récupéré après la séparation d'au moins une partie de l'hydrocarbure non oxydé, pour extraire les produits oxydés formés au cours de l'oxydation et solubles dans l'eau (produits non olonogènes lorsque l'hydrocarbure est le cyclohexane), et récupérer une phase organique comprenant, l'hydrocarbure non oxydé, l'hydroperoxyde d'alkyle et des produits oxydés non solubles dans l'eau (produits olonogènes lorsque l'hydrocarbure est le cyclohexane).

Selon l'invention, l'étape de lavage se caractérise en ce qu'elle est réalisée dans une colonne d'extraction liquide/liquide à contre courant, avec alimentation de l'eau de lavage en tête de colonne, du milieu réactionnel à traiter/laver à une position intermédiaire de la colonne, et en ce que de l'hydrocarbure saturé à oxyder, est alimenté dans la colonne de lavage à une position située en aval de la position d'alimentation du milieu à traiter/laver par rapport au sens de circulation de l'eau, c'est dire que de l'hydrocarbure saturé à oxyder, est alimenté dans la colonne de lavage à un niveau inférieur à celui de l'alimentation du milieu à laver.

Selon l'invention, l'hydrocarbure à oxyder est choisi dans le groupe comprenant le cyclohexane, le cyclooctane, le cyclododécane, la décaline. Le cyclohexane est l'hydrocarbure préféré. L'hydrocarbure alimenté dans l'opération de lavage est un hydrocarbure ne provenant pas d'une boucle de recyclage du procédé d'oxydation mais un hydrocarbure appelé « neuf » car ne contenant pas de produits oxydés et provenant de l'hydrocarbure approvisionné pour le procédé.

Selon une autre caractéristique de l'invention, le procédé d'oxydation peut être mis en oeuvre en absence de catalyseur ou en présence de catalyseur.

L'alimentation d'hydrocarbure tel que le cyclohexane dans la partie aval (inférieure) de la colonne d'extraction ou de lavage à l'eau, par rapport au sens de circulation de l'eau, permet d'extraire à partir de la phase aqueuse, dans cette partie aval de la colonne, les produits oxydés non solubles ou peu solubles dans l'eau. Ces produits sont notamment les composés oxydés olonogènes comme le cyclohexanol, la cyclohexanone, et l'hydroperoxyde de cyclohexyle.

Ainsi, l'alimentation d'hydrocarbure tel que le cyclohexane permet notamment de diminuer de manière très importante les concentrations en cyclohexanol, cyclohexanone, et hydroperoxyde de cyclohexyle dans la phase aqueuse effluente.

A titre indicatif, cette concentration en hydroperoxyde de cyclohexyle, cyclohexanol, ou cyclohexanone dans la phase aqueuse en sortie de colonne d'extraction est de l'ordre de plusieurs pour cent en poids pour chacun des composés sans alimentation en hydrocarbure dans la colonne de lavage. Le procédé de l'invention permet de réduire cette concentration à quelques centaines de ppm.

Un autre avantage de l'invention est de traiter par lavage aqueux au moins une partie de l'hydrocarbure neuf à oxyder avant son alimentation dans l'étape d'oxydation. En effet, les hydrocarbures transportés et stockés dans des containers métalliques peuvent contenir des impuretés métalliques telles des oxydes ou autres. Ces composés métalliques sont gênants dans le procédé d'oxydation du cyclohexane, notamment quand il est mis en oeuvre sans catalyseur pour produire de l'hydroperoxyde de cyclohexyle. En effet, la décomposition de l'hydroperoxyde de cyclohexyle est catalysée par les métaux et composés métalliques.

En conséquence, le procédé de production d'hydroperoxyde de cyclohexyle comprend avantageusement une étape de lavage aqueux du cyclohexane neuf pour éliminer ces composés métalliques avant alimentation du cyclohexane à l'étape d'oxydation.

Dans le procédé de l'invention, l'hydrocarbure neuf tel que le cyclohexane alimenté dans la colonne d'extraction permet d'une part d'extraire de la phase aqueuse les composés oxydés olonogènes peu solubles dans l'eau et d'autre part subit un lavage par l'eau avec une extraction des composés métalliques qui peuvent être contenus dans l'hydrocarbure.

L'hydrocarbure neuf ainsi lavé est introduit dans l'étape d'oxydation, de préférence avec l'hydrocarbure recyclé via les diverses étapes de recycle du procédé.

Le procédé de l'invention permet donc de réduire les pertes en produits oxydés valorisables par diminution de la concentration présente dans la phase aqueuse en sortie d'étape de lavage et également de diminuer la capacité de lavage aqueux à prévoir pour laver l'hydrocarbure avant l'alimentation dans l'étape d'oxydation.

L'étape de lavage du procédé de l'invention peut être mise en oeuvre dans tout dispositif convenable et est réalisée de préférence en continu.

Lorsque l'hydrocarbure considéré est le cyclohexane, le lavage est effectué à une température de 80 à 100 °C.

D'autres détails et avantages de l'invention apparaissent clairement au vu des exemples donnés ci-dessous uniquement à titre illustratif.

### Exemple 1 :

Dans une colonne de lavage comportant quarante plateaux réels et fonctionnant sous une pression de 2 bars, un mélange réactionnel provenant de l'oxydation par l'oxygène du cyclohexane mise en oeuvre en l'absence de catalyseur est alimenté en milieu de colonne. L'eau de lavage est alimentée en tête de colonne avec un débit correspondant à 5,5% en poids du flux de mélange réactionnel engagé dans la colonne. Le cyclohexane neuf (ne provenant pas d'un recyclage) est alimenté en pied de colonne avec un débit correspondant à 75% en poids du débit d'eau de lavage engagée en tête de colonne. L'effluent aqueux contenant 22% en poids de composés organiques est récupéré en fond de colonne. La concentration en produits olonogènes dans l'effluent aqueux est inférieure à 500 ppm (concentration en cyclohexanol/cyclohexanone voisine de 40 ppm, celle en hydroperoxyde de cyclohexyle voisine de 350 ppm).

Le lavage du milieu réactionnel est, selon le procédé de l'invention, réalisé avec une perte très faible en produits olonogènes.

### Exemple 2 :

Dans une colonne de lavage comportant quatre vingt plateaux réels et fonctionnant sous une pression de 2 bars, un mélange réactionnel provenant de l'oxydation par l'oxygène du cyclohexane mise en oeuvre en l'absence de catalyseur est alimenté en milieu de colonne. L'eau de lavage est alimentée en tête de colonne avec un débit correspondant à 5,1 % en poids de celui du mélange réactionnel à laver, toujours alimenté en milieu de colonne. Le cyclohexane neuf (ne provenant pas d'un recyclage) est alimenté en pied de colonne avec un débit correspondant à 70 % en poids du débit d'eau de lavage engagée en tête de colonne. L'effluent aqueux contenant 23 % en poids de composés organiques est récupéré en fond de colonne. La concentration en produits olonogènes dans l'effluent aqueux est inférieure à 1400 ppm (concentration en cyclohexanol / cyclohexanone inférieure à 50 ppm, celle en hydroperoxyde de cyclohexyle voisine de 1350 ppm). Le lavage du milieu réactionnel est, selon le procédé de l'invention, réalisé avec une perte très faible en produits olonogènes.

### Exemple 3 :

Dans une colonne de lavage comportant quatre vingt plateaux réels et fonctionnant sous une pression de 2 bars, un mélange réactionnel provenant de l'oxydation par l'oxygène du cyclohexane mise en oeuvre en l'absence de catalyseur est alimenté en milieu de colonne. L'eau de lavage est alimentée en tête de colonne avec un débit correspondant à 4,3 % en poids de celui du mélange réactionnel à laver, toujours alimenté en milieu de colonne. Le cyclohexane neuf (ne provenant pas d'un recyclage) est alimenté en pied de colonne avec un débit correspondant à 83 % en poids du débit d'eau de lavage engagée en tête de colonne. L'effluent aqueux contenant 19 % en poids de composés organiques est récupéré en fond de colonne. La concentration en produits olonogènes dans l'effluent aqueux est inférieure à 1600 ppm (concentration en cyclohexanol / cyclohexanone inférieure à 50 ppm, celle en hydroperoxyde de cyclohexyle voisine de 1500 ppm). Le lavage du milieu réactionnel est, selon le procédé de l'invention, réalisé avec une perte très faible en produits olonogènes.

### Exemple 4 :

Dans une colonne de lavage comportant quatre vingt plateaux réels et fonctionnant sous une pression de 2 bars, un mélange réactionnel provenant de l'oxydation par l'oxygène du cyclohexane mise en oeuvre en l'absence de catalyseur est alimenté en milieu de colonne. L'eau de lavage est alimentée en tête de colonne avec un débit correspondant à 4,7 % en poids de celui du mélange réactionnel à laver, toujours alimenté en milieu de colonne. Le cyclohexane neuf (ne provenant pas d'un recyclage) est alimenté en pied de colonne avec un débit correspondant à 82 % en poids du débit d'eau de lavage engagée en tête de colonne. L'effluent aqueux contenant 20 % en poids de composés organiques est récupéré en fond de colonne. La concentration en produits olonogènes dans l'effluent aqueux est inférieure à 1600 ppm (concentration en cyclohexanol / cyclohexanone inférieure à 50 ppm, celle en hydroperoxyde de cyclohexyle voisine de 1470 ppm). Le lavage du milieu réactionnel est, selon le procédé de l'invention, réalisé avec une perte très faible en produits olonogènes.

## Revendications

1. Procédé de fabrication d'hydroperoxyde d'alkyle par oxydation par l'oxygène moléculaire d'un hydrocarbure saturé comprenant les étapes successives suivantes :
- oxydation de l'hydrocarbure par l'oxygène,
- séparation à partir du milieu réactionnel, d'au moins une partie de l'hydrocarbure non oxydé et recyclage de celui-ci dans l'étape d'oxydation,
- lavage par de l'eau du milieu réactionnel d'oxydation récupéré après séparation d'au moins une partie de l'hydrocarbure saturé non oxydé, pour extraire les sous-produits oxydés solubles dans l'eau et récupérer une phase organique comprenant l'hydroperoxyde d'alkyle, de l'hydrocarbure non oxydé et des produits oxydés non solubles dans l'eau, **caractérisé en ce que** l'étape de lavage est réalisée dans une colonne d'extraction liquide/liquide à contre courant, avec alimentation de l'eau de lavage en tête de colonne et du milieu réactionnel à laver contenant l'hydroperoxyde d'alkyle à une position intermédiaire de la colonne, et **en ce que** de l'hydrocarbure saturé à oxyder est alimenté dans la colonne de lavage à un niveau inférieur à celui de l'alimentation du milieu à laver ;
le procédé étant **caractérisé en ce que** l'hydrocarbure est choisi dans le groupe comprenant le cyclohexane, le cyclooctane, le cyclododécane et la décaline et **en ce que** l'hydrocarbure alimenté dans l'opération de lavage est un hydrocarbure ne provenant pas d'une boucle de recyclage du procédé d'oxydation.

2. Procédé selon la revendication 1, **caractérisé en ce que** l'hydroperoxyde d'alkyle est l'hydroperoxyde de cyclohexyle, l'hydrocarbure saturé étant le cyclohexane.

3. Procédé selon la revendication 2, **caractérisé en ce que** le lavage est effectué à une température de 80 à 100°C.

## Patentansprüche

1. Verfahren zur Herstellung von Alkylhydroperoxid durch Oxidation eines gesättigten Kohlenwasserstoffs mit molekularem Sauerstoff, das die folgenden aufeinanderfolgenden Schritte umfasst:
- Oxidation des Kohlenwasserstoffs mit Sauerstoff,
- Abtrennung mindestens eines Teils des nicht oxidierten Kohlenwasserstoffs aus dem Reaktionsmedium und Rückführung des nicht oxidierten Kohlenwasserstoffs in den Oxidationsschritt,
- Waschen des nach Abtrennung mindestens eines Teils des nicht oxidierten gesättigten Kohlenwasserstoffs gewonnenen Oxidationsreaktionsmediums mit Wasser zum Extrahieren der wasserlöslichen oxidierten Nebenprodukte und zur Gewinnung einer organischen Phase, die das Alkylhydroperoxid, den nicht oxidierten Kohlenwasserstoff und nicht wasserlösliche oxidierte Produkte umfasst, **dadurch gekennzeichnet, dass** der Waschschritt in einer Flüssig/Flüssig-Gegenstromextraktionssäule unter Einspeisung des Waschwassers am Kopf der Säule und des zu waschenden Reaktionsmediums, das das Alkylhydroperoxid enthält, an einer intermediären Position der Säule durchgeführt wird und der zu oxidierende gesättigte Kohlenwasserstoff auf einer unter der Höhe der Einspeisung des zu waschenden Mediums liegenden Höhe in die Waschsäule eingespeist wird;
wobei das Verfahren **dadurch gekennzeichnet ist, dass** der Kohlenwasserstoff aus der Gruppe umfassend Cyclohexan, Cyclooctan, Cyclododecan und Decalin ausgewählt ist und es sich bei dem in den Wascharbeitsgang eingespeisten Kohlenwasserstoff um einen nicht aus einer Rückführungsschleife des Oxidationsverfahrens stammenden Kohlenwasserstoffs handelt.

2. Verfahren nach Anspruch 1, **dadurch gekennzeichnet, dass** es sich bei dem Alkylhydroperoxid um Cyclohexylhydroperoxid handelt, wobei es sich bei dem gesättigten Kohlenwasserstoff um Cyclohexan handelt.

3. Verfahren nach Anspruch 2, **dadurch gekennzeichnet, dass** das Waschen bei einer Temperatur von 80 bis 100°C durchgeführt wird.

## Claims

1. Process for the production of alkyl hydroperoxide by oxidation of a saturated hydrocarbon using molecular oxygen, comprising the following successive steps:
- oxidation of the hydrocarbon using oxygen,
- separation of at least a part of the nonoxidized hydrocarbon from the reaction medium and recycling of said nonoxidized hydrocarbon to the oxidation step,
- washing, with water, of the oxidation reaction medium recovered after the separation of at least a part of the nonoxidized saturated hydrocarbon, in order to extract the water-soluble oxidized by-products and to recover an organic phase comprising the alkyl hydroperoxide, the nonoxidized hydrocarbon and water-insoluble oxidized products, **characterized in that** the washing step is carried out in a counter-current liquid/liquid extraction column, with the washing water being fed at the top of the column and the reaction medium to be washed, containing the alkyl hydroperoxide, being fed at an intermediate position of the column, and **in that** the saturated hydrocarbon to be oxidized is fed into the washing column at a level below that at which the medium to be washed is fed;
the process being **characterized in that** the hydrocarbon is selected from the group consisting of cyclohexane, cyclooctane, cyclododecane and decalin and **in that** the hydrocarbon fed in the washing operation is a hydrocarbon which does not originate from a recycling loop of the oxidation process.

2. Process according to Claim 1, **characterized in that** the alkyl hydroperoxide is cyclohexyl hydroperoxide, the saturated hydrocarbon being cyclohexane.

3. Process according to Claim 2, **characterized in that** the washing is carried out at a temperature of from 80 to 100°C.
